Europäisches Patentamt

European Patent Office

Office européen des brevets

(11) Publication number: **0 387 216**
**A1**

## EUROPEAN PATENT APPLICATION

(21) Application number: **90830090.8**

(51) Int. Cl.⁵: **A61B 1/24**

(22) Date of filing: **08.03.90**

(30) Priority: **08.03.89 IT 4004289**

(43) Date of publication of application:
**12.09.90 Bulletin 90/37**

(84) Designated Contracting States:
**AT BE CH DE ES FR GB GR LI LU NL SE**

(71) Applicant: **Riccardi, Francesco**
**Via Pasubio, 3/0**
**I-43100 Parma(IT)**

Applicant: **Marzullo, Luigi**
**Via A. Frank, 8**
**I-43044 Collecchio (Parma)(IT)**

(72) Inventor: **Riccardi, Francesco**
**Via Pasubio, 3/0**
**I-43100 Parma(IT)**
Inventor: **Marzullo, Luigi**
**Via A. Frank, 8**
**I-43044 Collecchio (Parma)(IT)**

(74) Representative: **Lanzoni, Luciano**
**c/o BUGNION S.p.A. Viale Trento Trieste, 25**
**I-41100 Modena(IT)**

(54) **A dentist's mouth mirror.**

(57) In a mouth mirror, as used by dental surgeons, the hollow handle (10) is permanently attached to a cylindrical chamber (1) accommodating a vaned impeller (7) to which a circular reflector (9) is mounted, flush with the open top of the chamber; compressed air is directed through the handle into the chamber in order to rotate the impeller (7), and with it, the reflector (9).

fig.1

Xerox Copy Centre

The present invention relates to a mouth mirror of the type used by dentists.

Conventionally, a mouth mirror appears as a slender handle carrying a small round glass at one end. The plane occupied by the glass is set generally at an angle in relation to the axis of the handle, so as to facilitate inspection around all areas of the dentition.

In several types of dental treatment, the surgeon has to operate with a drill, or at all events with instruments that create debris by removing material from the teeth, while at the same time keeping the mirror in the patient's mouth to enable continued inspection of the treatment area.

In operating conditions such as these, it happens more often than not that the mirror is invested by debris or blood, with the result that visibility becomes adversely affected.

Thus, the need arises for the glass to be cleaned repeatedly.

A further drawback besetting the conventional mouth mirror is that the glass becomes clouded over with moisture from the patient's breath. Moreover in the interests of hygiene, the mirror must be sterilized before commencing treatment on each new patient. The object of the present invention is to prevent debris or blood deposits from forming on a mouth mirror during dental treatment, and thus to avoid the need for repeated cleaning of the mirror glass.

A further object of the invention is to prevent the glass of a mouth mirror from being clouded over by the patient's breath.

An additional object is to provide a low-cost mouth mirror that can be used once only and discarded on completion of the single patient's treatment.

The stated objects are comprehensively realized, according to the invention, in a mouth mirror as characterized in the appended claims.

The invention will now be described in detail, by way of example, with the aid of the accompanying drawings, in which:

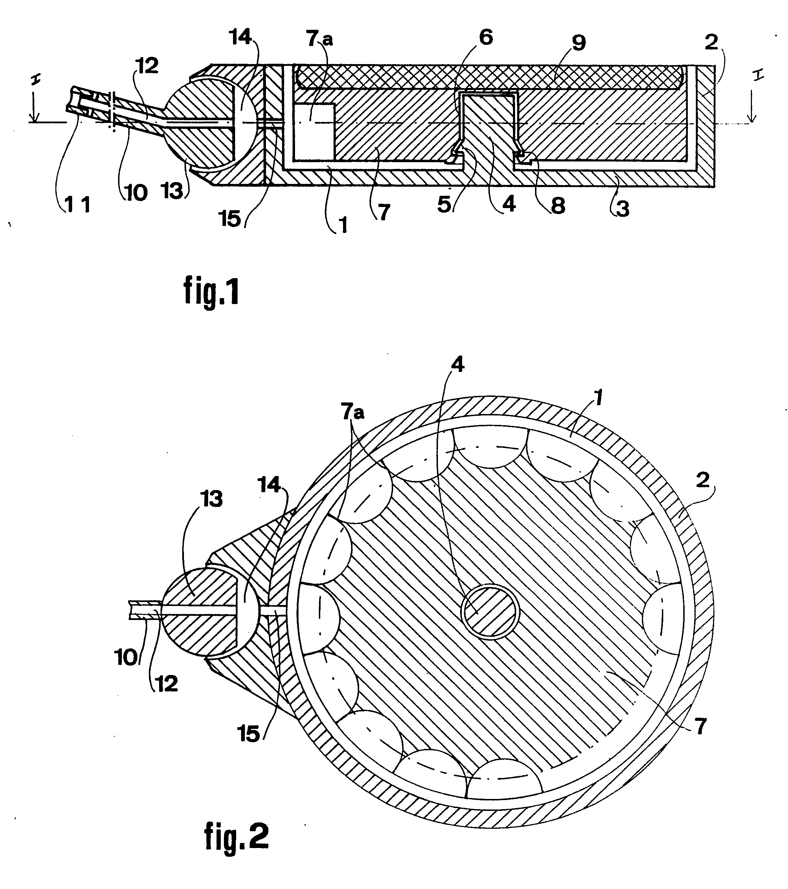

-fig 1 is a side elevation of the mirror, viewed in longitudinal section;

-fig 2 is a plan of the mirror viewed in section through I-I of fig 1.

With reference to the drawings, 1 denotes a chamber of cylindrical geometry formed by a side wall 2 and a bottom, or base 3.

The top of the cylindrical chamber remains open.

4 denotes a pivot integral with the base 3, which is disposed coaxially with the chamber and provided with a peripheral ring 5 of triangular section.

6 denotes the hub of an impeller 7 fitted over the pivot 4 and carrying a plurality of vanes 7a; the hub 6 presents an annular seating 8 designed to snap into positive engagement with the peripheral ring 5.

9 denotes a circular reflecting glass mounted to the surface of the impeller 7 uppermost.

The handle 10 of the mirror, which can be connected to a compressed air supply line 11, incorporates a duct 12 extending through a ball end 13 and into a spherical socket 14 that communicates with the cylindrical chamber 1 by way of an inlet 15 located in the side wall.

The ball-and-socket arrangement permits of angling the glass in any given direction.

The vanes 7a of the impeller 7 can thus be invested with a jet of compressed air supplied through the inlet 15, and the impeller set in rotation.

The speed of rotation of the impeller, hence of the mirror mounted to its top surface, is such that any particles of debris that may be deposited on the glass will be distanced forcibly by the resulting centrifugal force.

Alternative embodiments of the invention might feature a different preferred shape of the impeller and vanes, as also the joint between the handle and the cylindrical chamber might be other than that described and illustrated, or even rigid, without prejudice to the substance of the claims appended.

## Claims

1) A dentist's mouth mirror,
characterized
in that it comprises an open-topped cylindrical chamber (1) accommodating a vaned impeller (7) to which a circular reflecting glass (9) is mounted substantially flush with the open top of the chamber; and
in that the cylindrical chamber (1) is provided with an inlet (15) admitting compressed air which by impinging on the vanes of the impeller causes both impeller and glass to be set in rotation.

2) A mouth mirror as in claim 1, comprising a hollow handle (10) connected to a supply of compressed air and communicating with the cylindrical chamber (1) accommodating the impeller.

3) A mouth mirror as in claim 1, comprising a hollow handle (10) connected to a supply of compressed air and fitted to the cylindrical chamber (1) by way of a ball and socket joint (13, 14) so as to permit of angling the chamber, hence the glass (9), in any given direction, wherein the ball (13) affords a duct enabling the passage of compressed air from the handle to the cylindrical chamber.

4) A mouth mirror as in claim 1, wherein the impeller is rotatable about a pivot (4) associated coaxially with the cylindrical chamber (1), and provided with a hub (6) affording an annular seating (8) designed to snap into positive engagement over a peripheral ring (5) afforded by the pivot (4).

fig.1

fig.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-942100 (SIC PRODUCTS LTD.) <br> * page 2, lines 36 - 59 * | 1, 2, 4 | A61B1/24 |
| Y | * page 2, lines 74 - 120; figures 1, 3-6 * | 3 | |
| Y | CH-A-245535 (E. HAMBERGER) <br> * page 2, lines 6 - 32; figures * | 3 | |

TECHNICAL FIELDS SEARCHED (Int. Cl.5)

A61B

The present search report has been drawn up for all claims

| Place of search | Date of completion of the search | Examiner |
|---|---|---|
| THE HAGUE | 11 JUNE 1990 | RIEB K.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)